# EUROPÄISCHE PATENTSCHRIFT

(11) **EP 0 934 301 B1**
(45) Veröffentlichungstag und Bekanntmachung des Hinweises auf die Patenterteilung: **20.06.2001**
(21) Anmeldenummer: 97912140.7
(22) Anmeldetag: 08.10.1997
(51) Int. Cl.: C07D 333/68, C07D 333/38, C07D 409/12, C07D 333/78, C07D 333/80, A61K 31/38

(54) **AMINOTHIOPHENCARBONSÄUREAMIDE UND IHRE VERWENDUNG ALS PHOSPHODIESTERASE INHIBITOREN**
AMINOTHIOPHENE CARBOXYLIC ACID AMIDES AND THE USE THEREOF AS PHOSPHODIESTERASE INHIBITORS
AMIDES D'ACIDE CARBOXYLIQUE D'AMINOTHIOPHENE ET LEUR UTILISATION COMME INHIBITEURS DE PHOSPHODIESTERASE

(30) Priorität: 15.10.1996 DE 19642451
(43) Veröffentlichungstag der Anmeldung: 11.08.1999
(73) Patentinhaber: MERCK PATENT GmbH, 64293 Darmstadt (DE)
(72) Erfinder: JONAS, Rochus, D-64291 Darmstadt (DE); SCHELLING, Pierre, D-64367 Mühltal (DE); KLUXEN, Franz-Werner, D-64285 Darmstadt (DE); CHRISTADLER, Maria, D-63322 Rödermark (DE)
(86) Internationale Anmeldenummer: EP9705531
(87) Internationale Veröffentlichungsnummer: WO9816521

(56) Entgegenhaltungen:
- EP-A- 0 349 239
- EP-A- 0 623 607
- EP-A- 0 685 475
- DE-A- 4 230 755
- HUSSEIN F. ZOHDI ET. AL.: "Convenient heterocyclisation reactions with ethyl 2-amino-4,5,6,7- tetrahydrobenzo[b]thiophene- 3-carboxylate." JOURNAL OF CHEMICAL RESEARCH, SYNOPSIS, Nr. 10, Oktober 1996, Seiten 440-1, XP002055001
- F. SAUTER ET. AL. : "Neue Derivate der 2-Acylamino-thiophen (und benzo[b]thiophen)-3-carbonsäure sowie des ([1]Benzo-)thieno[2,3-d]- pyrimidin-4-ons." MONATSCHRIFT DER CHEMIE, Bd. 107, Nr. 3, März 1976, Seiten 669-73, XP002055002

## Beschreibung

Die Erfindung betrifft Verbindungen der Formel I 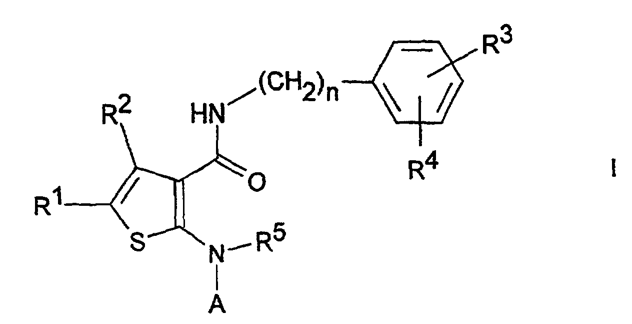 worin
- R¹, R²: jeweils unabhängig voneinander H, A, OA, Alkenyl, Alkinyl, CF₃ oder Hal,
wobei einer der Reste R¹ oder R² immer ≠ H ist,
- R¹ und R²: zusammen auch Alkylen mit 3-5 C-Atomen,
- R³, R⁴: jeweils unabhängig voneinander H, A, OA, NO₂, NH₂ oder Hal,
- R³ und R⁴: zusammen auch -O-CH₂-CH₂-, -O-CH₂-O- oder -O-CH₂-CH₂-O-,
- A, A': jeweils unabhängig voneinander H oder Alkyl mit 1 bis 6 C-Atomen,
- R⁵: -X-Y,
- X: CO, CS oder SO₂,
- Y: einen unsubstituierten oder ein- oder zweifach durch COOH, COOA, CONH₂, CONAA', CONHA, CN, NHSO₂A, N(SO₂A)₂ oder SO₂A substituierten gesättigten oder ungesättigten 5-7-gliedrigen isocyclischen Ring,
- Hal: F, Cl, Br oder I und
- n: 0, 1, 2 oder 3
bedeuten,
sowie deren physiologisch unbedenklichen Salze,
wobei 2-Benzoyl-4,5,6,7-tetrahydro-benzo[b]thiophen-3-carbonsäure N-phenylamid ausgeschlossen ist.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.

Insbesondere zeigen sie eine spezifische Inhibierung der cGMP-Phosphodiesterase (PDE V).

Chinazoline mit cGMP-Phosphodiesterase hemmender Aktivität sind z.B. in J. Med. Chem. 36, 3765 (1993) und ibid. 37, 2106 (1994) beschrieben. Pyrazolopyrimidone, die zur Behandlung von Potenzstörungen geeignet sind, sind z.B in der WO 94/28902 beschrieben.

Andere Tetrahydrobenzo[b]thiophenderivate sind z.B. beschrieben in J. Chem. Research (S), 1996, 440-441.

Thienopyrimidinderivate unterschiedlicher Struktur kennt man z.B. aus EP 0 349 239 als PDE V-Hemmer.

Die biologische Aktivität der Verbindungen der Formel I kann nach Methoden bestimmt werden, wie sie z.B in der WO 93/06104 oder der WO 94/28902 beschrieben sind.
Die Affinität der erfindungsgemäßen Verbindungen für cGMP- und cAMP-Phosphodiesterase wird durch die Ermittlung ihrer IC₅₀-Werte (Konzentration des Inhibitors, die benötigt wird, um eine 50 %ige Inhibierung der Enzymaktivität zu erreichen) bestimmt
Zur Durchführung der Bestimmungen können nach bekannten Methoden isolierte Enzyme verwendet werden (z.B. W.J. Thompson et al., Biochem. 1971, 10, 311). Zur Durchführung der Versuche kann eine modifizierte "batch"-Methode von W.J. Thompson und M.M. Appleman (Biochem. 1979, 18, 5228) angewendet werden.

Die Verbindungen eignen sich daher zur Behandlung von Erkrankungen des Herz-Kreislaufsystems, insbesondere der Herzinsuffizienz, und zur Therapie von Potenzstörungen.

Die Verbindungen der Formel I können als Arzneimittelwirkstoffe in der Human- und Veterinärmedizin eingesetzt werden. Femer können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zur Herstellung von Verbindungen der Formel I nach Anspruch 1 sowie deren Salzen,
dadurch gekennzeichnet, daß man
a) eine Verbindung der Formel II 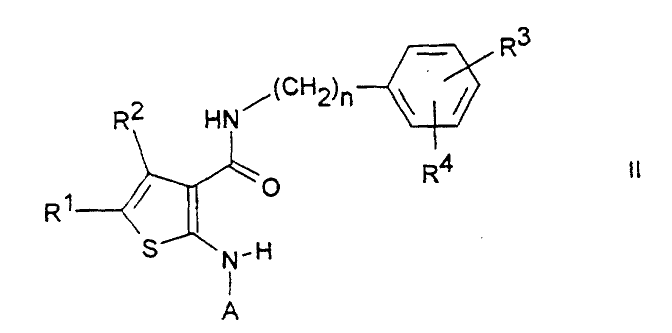 worin
   R¹, R², R³, R⁴ und n die angegebenen Bedeutungen haben,
   mit einer Verbindung der Formel III

   L-R⁵ III

   umsetzt,
   worin
   R⁵ die angegebene Bedeutung hat und
   L Cl, Br, I, OH oder eine reaktionsfähig veresterte OH-Gruppe bedeutet,
   oder
b) eine Verbindung der Formel IV 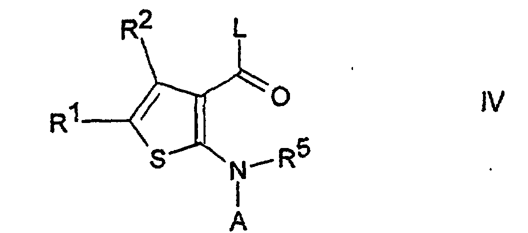 worin
   R¹, R², R⁵ und A die angegebenen Bedeutungen haben,
   und L Cl, Br, I, OH oder eine reaktionsfähig veresterte OH-Gruppe bedeutet,
   mit einer Verbindung der Formel V 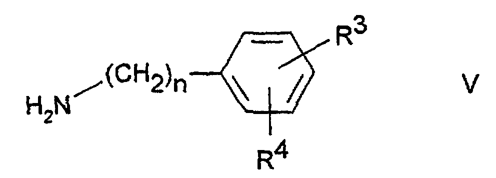 worin
   R³, R⁴ und n die angegebenen Bedeutungen haben,
   umsetzt,
   oder
c) in einer Verbindung der Formel I einen Rest R³, R⁴ und/oder R⁵ in einen anderen Rest R³, R⁴ und/oder R⁵ umwandelt, indem man einen Ester verseift oder eine Nitrogruppe reduziert,
   und/oder daß man eine Säure der Formel I durch Behandeln mit einer Base in eines ihrer Salze überführt.

Vor- und nachstehend haben die Reste R¹, R², R³, R⁴, R⁵, A, L und n die bei den Formeln I, II, III, IV und V angegebenen Bedeutungen, sofern nicht ausdrücklich etwas anderes angegeben ist.

A und A' bedeuten vorzugsweise jeweils unabhängig voneinander H oder Alkyl mit 1-6 C-Atomen.

In den vorstehenden Formeln ist Alkyl vorzugsweise unverzweigt und hat 1, 2, 3, 4, 5 oder 6 C-Atome, vorzugsweise 1, 2, 3, 4 oder 5 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, Neopentyl oder Isopentyl.

Alkylen ist vorzugsweise unverzweigt und bedeutet bevorzugt Propylen, Butylen oder Pentylen.

Von den Resten R¹ und R² steht einer vorzugsweise für H, während der andere bevorzugt Propyl oder Butyl, besonders bevorzugt aber Ethyl oder Methyl bedeutet. Ferner bedeuten R¹ und R² auch zusammen bevorzugt Propylen, Butylen oder Pentylen.

Hal bedeutet vorzugsweise F, Cl oder Br, aber auch I.

Alkenyl steht vorzugsweise für Vinyl, 1- oder 2-Propenyl, 1-Butenyl, lsobutenyl, sek.-Butenyl, ferner bevorzugt ist 1-Pentenyl, iso-Pentenyl oder 1-Hexenyl.

Alkinyl steht vorzugsweise für Ethinyl, Propin-1-yl, ferner für Butin-1-, Butin-2-yl, Pentin-1-, Pentin-2- oder Pentin-3-yl.

Die Reste R³ und R⁴ können gleich oder verschieden sein und stehen vorzugsweise in der 3- oder 4-Position des Phenylrings. Sie bedeuten beispielsweise jeweils unabhängig voneinander H, Alkyl, Alkoxy, Nitro, Amino, Alkylamino wie z.B. Methylamino, Dialkylamino wie z.B. Dimethylamino, F, Cl, Br oder I oder zusammen Ethylenoxy, Methylendioxy oder Ethylendioxy. Bevorzugt stehen sie auch jeweils für Alkoxy, wie z.B. für Methoxy, Ethoxy oder Propoxy.

Der Rest Y ist vorzugsweise unsubstituiertes oder ein- oder zweifach durch COOH, COOCH₃,COOC₂H₅, CONH₂, CON(CH₃)₂, CONHCH₃, CN, NHSO₂CH₃, N(SO₂CH₃)₂ oder SO₂CH₃ substituiertes Cyclopentyl, Cyclohexyl, Cycloheptyl, Phenyl, 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazotyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl.

Insbesondere bedeutet Y z.B. 4-Methoxycarbonylphenyl, 4-Carboxyphenyl, 4-Methoxycarbonylcyclohexyl, 4-Carboxycyclohexyl, 4-Methylsulfonamidophenyl, 4-Methylsulfonamidocyclohexyl, 4-Aminocarbonylphenyl oder 4-Aminocarbonylcyclohexyl.

X bedeutet vorzugsweise CO, ferner auch CS oder SO₂.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis le ausgedrückt werden, die der Formel I entsprechen und worin die nicht näher bezeichneten Reste die bei der Formel I angegebene Bedeutung haben, worin jedoch
- in la Y: einen unsubstituierten oder ein- oder zweifach durch COOH, COOA, CONH₂, CONAA', CONHA, CN, NHSO₂A, N(SO₂A)₂ oder SO₂A substituierten Phenyl- oder Cyclohexylring bedeutet;
- in Ib R¹, R²: jeweils unabhängig voneinander H, A, OA, NO₂, CF₃ oder Hal,
wobei mindestens einer der Reste R¹ oder R² immer ≠ H ist.
- R³ und R⁴: zusammen -O-CH₂-CH₂-, -O-CH₂-O- oder -O-CH₂-CH₂-O.
- X: CO,
- Y: einen unsubstituierten oder ein- oder zweifach durch COOH, COOA, CONH₂, CONAA', CONHA, CN, NHSO₂A, N(SO₂A)₂ oder SO₂A substituierten Phenyl- oder Cydohexylring und
- n: 1 bedeuten;
- in lc R¹, R²: jeweils unabhängig voneinander H, A, OA, NO₂, CF₃ oder Hal,
wobei mindestens einer der Reste R¹ oder R² immer ≠ H ist,
- R³, R⁴: jeweils unabhängig voneinander H, A, OA, Hal, NO₂, oder NH₂,
- X: CO,
- Y: einen unsubstituierten oder ein- oder zweifach durch COOH, COOA, CONH₂, CONAA', CONHA, CN, NHSO₂A, N(SO₂A)₂ oder SO₂A substituierten Phenyl- oder Cyclohexylring und
- n: 1 bedeuten;
- in Id R¹ und R²: zusammen Alkylen mit 3-5 C-Atomen,
- R³ und R⁴: zusammen -O-CH₂-CH₂-, -O-CH₂-O- oder -O-CH₂-CH₂-O,
- X: CO,
- Y: einen unsubstituierten oder ein- oder zweifach durch COOH, COOA, CONH₂, CONAA', CONHA, CN, NHSO₂A, N(SO₂A)₂ oder SO₂A substituierten Phenyl- oder Cydohexylring und
- n: 1 bedeuten;
- in le R¹ und R²: zusammen Alkylen mit 3-5 C-Atomen,
- R³, R⁴: jeweils unabhängig voneinander H, A, OA, Hal, NO₂, oder NH₂,
- X: CO,
- Y: einen unsubstituierten oder ein- oder zweifach durch COOH, COOA, CONH₂, CONAA', CONHA, CN, NHSO₂A, N(SO₂A)₂ oder SO₂A substituierten Phenyl- oder Cyclohexylring und
- n: 1 bedeuten.

Die Verbindungen der Formel I und auch die Ausgangsstoffe zu ihrer Hersteffung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II, III, IV und V haben R¹, R², R³, R⁴, R⁵ und n die angegebenen Bedeutungen, insbesondere die angegebenen bevorzugten Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyloder p-Tolylsulfonyloxy, ferner auch 2-Naphthalinsulfonyloxy).

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt. Andererseits ist es möglich, die Reaktion stufenweise durchzuführen,

Die Verbindungen der Formel I können vorzugsweise erhalten werden, indem man Verbindungen der Formel II mit Verbindungen der Formel III umsetzt.

Die Ausgangsstoffe der Formeln II sind teilweise bekannt. Sofern sie nicht bekannt sind, können sie nach an sich bekannten Methoden hergestellt werden. Die Amide der Formel II sind nach Houben-Weyl E6a, 320 aus Aldehyden bzw. Ketonen und substituierten Cyanacetamiden in Gegenwart von Schwefel erhältlich.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Verbindungen der Formel I können weiterhin erhalten werden, indem man Verbindungen der Formel IV mit Verbindungen der Formel V umsetzt.
Die Ausgangsverbindungen der Formel IV und V sind in der Regel bekannt Sind sie nicht bekannt, so können sie nach an sich bekannten Methoden hergestellt werden.
Die Umsetzung der Verbindungen der Formel IV mit Verbindungen der Formel V erfolgt unter ähnlichen Bedingungen, betreffend die Reaktionszeit, Temperatur und Lösungsmittel, wie dies für die Umsetzung der Verbindungen der Formel II mit den Verbindungen der Formel III beschrieben ist.

Es ist ferner möglich, in einer Verbindung der Formel I einen Rest R³ und/oder R⁴ in einen anderen Rest R³ und/oder R⁴ umzuwandeln, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert oder Cyangruppen zu COOH-Gruppen hydrolysiert. COOA-Gruppen können z.B. mit NaOH oder KOH in Wasser, Wasser-THF oder Wasser-Dioxan bei Temperaturen zwischen 0 und 100° verseift werden.
Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Säure der Formel I kann mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann die Säure der Formel I mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.

Andererseits kann eine Base der Formel I mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefem. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, femer organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzofsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der Formel I und ihre physiologisch unbedenklichen Salze als Phosphodiesterase V-Hemmer.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen konnen stermsiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cGMP(cyclo-Guanosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Krankheiten des HerzKreislaufsystems und zur Therapie von Potenzstörungen finden.

Dabei werden die Substanzen in der Regel vorzugsweise in Dosierungen zwischen etwa 1 und 500 mg, insbesondere zwischen 5 und 100 mg pro Dosierungseinheit verabreicht Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.
- Massenspektrometrie (MS):: El (Elektronenstoß-lonisation) M⁺
FAB (Fast Atom Bombardment) (M+H)⁺

### Beispiel 1

Eine Lösung von 1,5 g 2-Amino-4,5,6,7-tetrahydrobenzo[b]thiophen-3-carbonsäure-(benzo[1,3]dioxol-5-ylmethyl)-amid [erhältlich durch Umsetzung von Cyclohexanon mit N-Benzo[1,3]dioxol-5-ylmethyl-2-cyanoacetamid in Gegenwart von Schwefel] in 50 ml Dichlormethan und 2 ml Pyridin wird mit 1,0 g Methoxycarbonylbenzoylchlorid ("A") versetzt und 2 Stunden bei Raumtemperatur gerührt. Das Lösungsmittel wird entfernt und wie üblich aufgearbeitet. Man erhält 1,3 g 4-{3-[(Benzo[1,3]-dioxol-5-ylmethyl)-carbamoyl]-4,5,6,7-tetrahydro-benzo[b]thiophen-2-ylcarbamoyl}benzoesäuremethylester, F. 165°.

Analog erhält man durch Umsetzung von "A"
mit 2-Amino-5-methyl-thiophen-3-carbonsäure-(benzo[1,3]dioxol-5-yl-methyl)-amid
   4-{3-[(Benzo[1,3]-dioxol-5-ylmethyl)-carbamoyl]-5-methyl-thiophen-2-ylcarbamoyl}-benzoesäuremethylester, F. 138°;
mit 2-Amino-5,6-dimethyl-thiophen-3-carbonsäure-(benzo[1,3]dioxol-5-yl-methyl)-amid
   4-{3-[(Bezno[1,3]-dioxol-5-ylmethyl)-carbamoyl]-5,6-dimethylthiophen-2-ylcarbamoyl}-benzoesäuremethylester
mit 2-Amino-5-chlor-thiophen-3-carbonsäure-(benzo[1,3]dioxol-5-ylmethyl)amid
   4-{3-[(Benzo[1,3]-dioxol-5-ylmethyl)-carbamoyl]-5-chlor-thiophen-2-ylcarbamoyl}-benzoesäuremethylester;
mit 2-Amino-4,5-cyclopenteno-thiophen-3-carbonsäure-(benzo[1,3]dioxol-5-ylmethyl)-amid
   4-{3-[(Benzo[1,3]-dioxol-5-ylmethyl)-carbamoyl]-4,5-cyclopentenothiophen-2-ylcarbamoyl}-benzoesäuremethylester;
mit 2-Amino-4,5-cyclohepteno-thiophen-3-carbonsäure-(benzo[1,3]dioxol-5-ylmethyl)-amid
   4-{3-[(Benzo[1,3]-dioxol-5-ylmethyl)-carbamoyl]-4,5-cycloheptenothiophen-2-ylcarbamoyl}-benzoesäuremethylester;
mit 2-Amino-5-ethyl-thiophen-3-carbonsäure-(benzo[1,3]dioxol-5-ylmethyl)-amid
   4-{3-[(Benzo[1,3]-dioxol-5-ylmethyl)-carbamoyl]-5-ethyl-thiophen-2-ylcarbamoyl}-benzoesäuremethylester;
mit 2-Amino-5-propyl-thiophen-3-carbonsäure-(benzo[1,3]dioxol-5-yl-methyl)-amid
   4-{3-[(Benzo[1,3]-dioxol-5-ylmethyl)-carbamoyl]-5-propyl-thiophen-2-ylcarbamoyl}-benzoesäuremethylester;
mit 2-Amino-5-isopropyl-thiophen-3-carbonsäure-(benzo[1,3]dioxol-5-yl-methyl)-amid
   4-{3-[(Benzo[1,3]-dioxol-5-ylmethyl)-carbamoyl]-5-isopropyl-thiophen-2-ylcarbamoyl}-benzoesäuremethylester;
mit 2-Amino-5-butyl-thiophen-3-carbonsäure-(benzo[1,3]dioxol-5-ylmethyl)-amid
   4-{3-[(Benzo[1,3J-dioxol-5-ylmethyl)-carbamoyl]-5-butyl-thiophen-2-ylcarbamoyl}-benzoesäuremethylester;
mit 2-Amino-4,5,6,7-tetrahydrobenzo[b]thiophen-3-carbonsäure-N-benzyl-amid
   4-[3-(Benzyl-carbamoyl)-4,5,6,7-tetrahydro-benzo[b]thiophen-2-yl-carbamoyl]-benzoesäuremethylester
mit 2-Amino-5-methyl-thiophen-3-carbonsäure-N-benzylamid
   4-[3-(Benzyl-carbamoyl)-5-methyl-thiophen-2-ylcarbamoyl]-benzoesäuremethylester, F. 170°;
mit 2-Amino-5-isopropyl-thiophen-3-carbonsäure-N-benzylamid
   4-[3-(Benzyl-carbamoyl)-5-isopropyl-thiophen-2-ylcarbamoyl]-benzoesäuremethylester, F. 170-172°;
mit 2-Amino-5-chlor-thiophen-3-carbonsäure-N-benzylamid
   4-[3-(Benzyl-carbamoyl)-5-chlor-thiophen-2-ylcarbamoyl]-benzoesäuremethylester;
mit 2-Amino-4,5-cyclopenteno-thiophen-3-carbonsäure-N-benzylamid
   4-[3-(Benzyl-carbamoyl)-4,5-cyclopenteno-thiophen-2-ylcarbamoyl]-benzoesäuremethylester;
mit 2-Amino-4,5-cyclohepteno-thiophen-3-carbonsäure-N-benzylamid
   4-[3-(Benzyl-carbamoyl)-4,5-cyclohepteno-thiophen-2-ylcarbamoyl]-benzoesäuremethylester;
mit 2-Amino-5-ethyl-thiophen-3-carbonsäure-N-benzylamid
   4-[3-(Benzyl-carbamoyl)-5-ethyl-thiophen-2-ylcarbamoyl]-benzoesäuremethylester;
mit 2-Amino-4,5,6,7,-tetrahydrobenzo[b]thiophen-3-carbonsäure-N-(3-chlor-4-methoxybenzyl)-amid
   4-{3-[(3-Chlor-4-methoxybenzyl)-carbamoyl]-4,5,6,7-tetrahydrobenzo[b]thiophen-2-ylcarbamoyl}-benzoesäuremethylester;
mit 2-Amino-5-methyl-thiophen-3-carbonsäure-N-(3-chlor-4-methoxybenzyl)-amid
   4-{3-[(3-Chlor-4-methoxybenzyl)-carbamoyl]-5-methyl-thiophen-2-yl-carbamoyl}-benzoesäuremethylester;
mit 2-Amino-5-chlor-thiophen-3-carbonsäure-N-(3-chlor-4-methoxybenzyl)-amid
   4-{3-[(3-Chlor-4-methoxybenzyl)-carbamoyl]-5-chlor-thiophen-2-yl-carbamoyl}-benzoesäuremethylester;
mit 2-Amino-4,5-cyclopenteno-thiophen-3-carbonsäure-N-(3-Chlor-4-methoxybenzyl)-amid
   4-{3-[(3-Chlor-4-methoxybenzyl)-carbamoyl]-4,5-cyclopenteno-thiophen-2-ylcarbamoyl}-benzoesäuremethylester;
mit 2-Amino-4,5-cyclohepteno-thiophen-3-carbonsäure-N-(3-Chlor-4-methoxybenzyl)-amid
   4-{3-[(3-Chlor-4-methoxybenzyl)-carbamoyl]-4,5-cycloheptenothiophen-2-ylcarbamoyl}-benzoesäuremethylester;
mit 2-Amino-5-ethyl-thiophen-3-carbonsäure-N-(3-chlor-4-methoxybenzyl)-amid
   4-{3-[(3-Chlor-4-methoxybenzyl)-carbamoyl]-5-ethyl-thiophen-2-yl-carbamoyl}-benzoesäuremethylester;
mit 2-Amino-4,5,6,7-tetrahydrobenzo[b]thiophen-3-carbonsäure-N-(3,4-dimethoxybenzyl)-amid
   4-{3-[(3,4-Dimethoxybenzyl)-carbamoyl]-4,5,6,7-tetrahydrobenzo[b]thiophen-2-ylcarbamoyl}-benzoesäuremethylester;
mit 2-Amino-5-methyl-thiophen-3-carbonsäure-N-(3,4-dimethoxybenzyl)-amid
   4-{3-[(3,4-Dimethoxybenzyl)-carbamoyl]-5-methyl-thiophen-2-yl-carbamoyl}-benzoesäuremethylester, F. 175°;
mit 2-Amino-5-chlor-thiophen-3-carbonsäure-N-(3,4-dimethoxybenzyl)-amid
   4-{3-[(3,4-Dimethoxybenzyl)-carbamoyl]-5-chlor-thiophen-2-yl-carbamoyl}-benzoesäuremethylester;
mit 2-Amino-4,5-cyclopenteno-thiophen-3-carbonsäure-N-(3,4-dimethoxybenzyl)-amid
   4-{3-[(3,4-Dimethoxybenzyl)-carbamoyl]-4,5-cyclopenteno-thiophen-2-ylcarbamoyl}-benzoesäuremethylester;
mit 2-Amino-4,5-cyclohepteno-thiophen-3-carbonsäure-N-(3,4-dimethoxybenzyl)-amid
   4-{3-[(3,4-Dimethoxybenzyl)-carbamoyl]-4,5-cyclohepteno-thiophen-2-ylcarbamoyl}-benzoesäuremethylester;
mit 2-Amino-5-ethyl-thiophen-3-carbonsäure-N-(3,4-dimethoxybenzyl)-amid
   4-{3-[(3,4-Dimethoxybenzyl)-carbamoyl]-5-ethyl-thiophen-2-yl-carbamoyl}-benzoesäuremethylester;
mit 2-Amino-4,5,6,7-tetrahydrobenzo[b]thiophen-3-carbonsäure-N-(4-fluorbenzyl)-amid
   4-{3-[(4-Fluorbenzyl)-carbamoyl]-4,5,6,7-tetrahydrobenzo[b]thiophen-2-ylcarbamoyl}-benzoesäuremethylester;
mit 2-Amino-5-methyl-thiophen-3-carbonsäure-N-(4-fluorbenzyl)-amid
   4-{3-[(4-Fluorbenzyl)-carbamoyl]-5-methyl-thiophen-2-ylcarbamoyl}-benzoesäuremethylester;
mit 2-Amino-5-chfor-hhiophen-3-carbonsäure-N-(4-fluorbenzyl)-amid
   4-{3-[(4-Fluorobenzyl)-carbamoyl]-5-chlor-thiophen-2-ylcarbamoyl}-benzoesäuramethpester;
mit 2-Amino-4,5-cyclopenteno-thiophen-3-carbonsäure-N-(4-fluorbenzyl)-amid
   4-{3-[(4-Fluorbenzyl)-carbamoyl]-4,5-cyclopenteno-thiophen-2-yl-carbamoyl}-benzoesäuremethylester;
mit 2-Amino-4,5-cyclohepteno-thiophen-3-carbonsäure-N-(4-fluorbenzyl)-amid
   4-{3-[(4-Fluorbenzyl)-carbamoyl]-4,5-cyclohepteno-thiophen-2-yl-carbamoyl}-benzoesäuremethylester;
mit 2-Amino-5-ethyl-thiophen-3-carbonsäure-N-(4-fluorbenzyl)-amid
   4-{3-[(4-Fluorbenzyl)-carbamoyl]-5-ethyl-thiophen-2-ylcarbamoyl}-benzoesäuremethylester;
mit 2-Amino-4,5,6,7-tetrahydrobenzo[b]thiophen-3-carbonsäure-N-(3-nitrobenzyl)-amid
   4-{3-[(3-Nitrobenzyl)-carbamoyl]-4,5,6,7-tetrahydro-benzo[b]thiophen-2-ylcarbamoyl}-benzoesäuremethylester;
mit 2-Amino-5-methyl-thiophen-3-carbonsäure-N-(3-nitrobenzyl)-amid
   4-{3-[(3-Nitrobenzyl)-carbamoyl]-5-methyl-thiophen-2-ylcarbamoyl}-benzoesäuremethylester;
mit 2-Amino-5-Chlor-thiophen-3-carbonsäure-N-(3-nitrobenzyl)-amid
   4-{3-[(3-Nitrobenzyl)-carbamoyl]-5-chlor-thiophen-2-ylcarbamoyl}-benzoesäuremethylester;
mit 2-Amino-4,5-cyclopenteno-thiophen-3-carbonsäure-N-(3-nitrobenzyl)-amid
   4-{3-[(3-Nitrobenzyl)-carbamoyl]-4,5-cyclopenteno-thiophen-2-yl-carbamoyl}-benzoesäuremethylester;
mit 2-Amino-4,5-cyclohepteno-thiophen-3-carbonsäure-N-(3-nitrobenzyl)-amid
   4-{3-[(3-Nitrobenzyl)-carbamoyl]-4,5-cyclohepteno-thiophen-2-yl-carbamoyl}-benzoesäuremethylester;
mit 2-Amino-5-ethyl-thiophen-3-carbonsäure-N-(3-nitrobenzyl)-amid
   4-{3-[(3-Nitrobenzyl)-carbamoyl]-5-ethyl-thiophen-2-ylcarbamoyl}-benzoesäuremethylester;
mit 2-Amino-4,5,6,7-tetrahydrobenzo[b]thiophen-3-carbonsäure-N-phenethylamid
   4-[3-(Phenethyl-carbamoyl)-4,5,6,7-tetrahydro-benzo[b]thiophen-2-yl-carbamoyl]-benzoesäuremethylester;
mit 2-Amino-5-methyl-thiophen-3-carbonsäure-N-phenethylamid
   4-[3-(Phenethyl-carbamoyl)-5-methyl-thiopheo-2-ylcarbamoyl]-benzoesäuremethylester;
mit 2-Amino-5-chlor-thiophen-3-carbonsäure-N-phenethylamid
   4-[3-(Phenethyl-carbamoyl)-5-chlor-thiophen-2-ylcarbamoyl]-benzoesäuremethylester;
mit 2-Amino-4,5-cyclopenieno-thiophen-3-carbonsäure-N-phenethylamid
   4-[3-(Phenethyl-carbamoyl)-4,5-cyclopenteno-thiophen-2-yl-carbamoyl]-benzoesäuremethylester;
mit 2-Amino-4,5-cyclohepteno-thiophen-3-carbonsäure-N-phenethylamid
   4-[3-(Phenethyl-carbamoyl)-4,5-cyclohepteno-thiophen-2-yl-carbamoyl]-benzoesäuremethylester;
mit 2-Amino-5-ethyl-thiophen-3-carbonsäure-N-phenethylamid
   4-[3-(Phenethyl-carbamoyl)-5-ethyl-thiophen-2-ylcarbamoyl]-benzoesäuremethylester.

### Beispiel 2

Analog Beispiel 1 erhält man durch Umsetzung von 2-Amino-4,5,6,7-tetrahydrobenzo[b]thiophen-3-carbonsäure-(benzo[1,3]dioxol-5-ylmethyl)-amid mit 4-Methoxycarbonyl-cyclohexancarbonsäurechlorid ("B") die Verbindung 4-{3-[(Benzo[1,3]-dioxol-5-ylmethyl)-carbamoyl]-4,5,6,7-tetrahydro-benzo[b]thiophen-2-ylcarbamoyl}-cyclohexancarbonsäuremethylester, F. 173°.

Analog erhält man durch Umsetzung von "B"
mit 2-Amino-5-methyl-thiophen-3-carbonsäure-(benzo[1,3]dioxol-5-yl-methyl)-amid
   4-{3-[(Benzo[1,3]-dioxol-5-ylmethyl)-carbamoyl]-5-methyl-thiophen-2-ylcarbamoyl}-cyclohexancarbonsäuremethylester, Öl;
mit 2-Amino-5,6-dimethyl-thiophen-3-carbonsäure-(benzo[1,3]dioxol-5-yl-methyl)-amid
   4-{3-[(Benzo[1,3]-dioxol-5-ylmethyl)-carbamoyl]-5,6-dimethyl-thiophen-2-ylcarbamoyl}-cyclohexancarbonsäuremethylester;
mit 2-Amino-5-chlor-thiophen-3-carbonsäure-(benzo[1,3]dioxol-5-ylmethyl)-amid
   4-{3-[(Benzo[1,3]-dioxol-5-ylmethyl)-carbamoyl]-5-chlor-thiophen-2-ylcarbamoyl}-cyclohexancarbonsäuremethylester;
mit 2-Amino-4,5-cyclopenteno-thiophen-3-carbonsäure-(benzo[1,3]dioxol-5-ylmethyl)-amid
   4-{3-[(Benzo[1,3]-dioxol-5-ylmethyl)-carbamoyl]-4,5-cyclopentenothiophen-2-ylcarbamoyl}-cyclohexancarbonsäuremethylester;
mit 2-Amino-4,5-cyclohepteno-thiophen-3-carbonsäure-(benzo[1,3]dioxol-5-ylmethyl)-amid
   4-{3-((Benzo[1,3]-dioxol-5-ylmethyl)-carbamoyl]-4,5-cycloheptenothiophen-2-ylcarbamoyl}-cyclohexancarbonsäuremethylester,
mit 2-Amino-5-ethyl-hhiophen-3-carbonsäure-(benzo[1,3]dioxol-5-ylmethyl)-amid
   4-{3-[(Benzo[1,3]-dioxol-5-ylmethyl)-carbamoyl]-5-ethyl-thiophen-2-ylcarbamoyl}-cyclohexancarbonsäuremethylester, Öl;
mit 2-Amino-4,5,6,7-tetrahydrobenzo[b]thiophen-3-carbonsäure-N-benzylamid
   4-[3-(Benzyl-carbamoyl)-4,5,6,7-tetrahydro-benzo[b]thiophen-2-yl-carbamoyl]-cyclohexancarbonsäuremethylester;
mit 2-Amino-5-methyl-thiophen-3-carbonsäure-N-benzylamid
   4-[3-(Benzyl-carbamoyl)-5-methyl-thiophen-2-ylcarbamoyl]-cyclohexancarbonsäuremethylester;
mit 2-Amino-5-chlor-thiophen-3-carbonsäure-N-benzylamid
   4-[3-(Benzyl-carbamoyl)-5-chlor-thiophen-2-ylcarbamoyl]-cyclohexancarbonsäuremethylester;
mit 2-Amino-4,5-cyclopenteno-thiophen-3-carbonsäure-N-benzylamid
   4-[3-(Benzyl-carbamoyl)-4,5-cyclopenteno-thiophen-2-ylcarbamoyl]-cyclohexancarbonsäuremethylester;
mit 2-Amino-4,5-cyclohepteno-thiophen-3-carbonsäure-N-benzylamid
   4-[3-(Benzyl-carbamoyl)-4,5-cyclohepteno-thiophen-2-ylcarbamoyl]-cyclohexancarbonsäuremethylester;
mit 2-Amino-5-ethyl-thiophen-3-carbonsäure-N-benzylamid
   4-[3-(Benzyl-carbamoyl)-5-ethyl-thiophen-2-ylcarbamoyl]-cyclohexancarbonsäuremethylester;
mit 2-Amino-4,5,6,7-tetrahydrobenzo[b]thlophen-3-carbonsäure-N-(3-chlor-4-methoxybenzyl)-amid
   4-{3-[(3-Chlor-4-methoxybenzyl)-carbamoyl]-4,5,6,7-tetrahydrobenzo[b]thiophen-2-ylcarbamoyl}-cyclohexancarbonsäuremethylester;
mit 2-Amino-5-methyl-thiophen-3-carbonsäure-N-(3-Chlor-4-methoxybenzyl)-amid
   4-{3-[(3-Chlor-4-methoxybenzyl)-carbamoyl]-5-methyl-thiophen-2-yl-carbamoyl}-cyclohexancarbonsäuremethylester;
mit 2-Amino-5-chlor-thiophen-3-carbonsäure-N-(3-chlor-4-methoxybenzyl)-amid
   4-{3-[(3-Chlor-4-methoxybenzyl)-carbamoyl]-5-chlor-thiophen-2-yl-carbamoyl}-cyclohexancarbonsäuremethylester;
mit 2-Amino-4,5-cyclopenteno-thiophen-3-carbonsäure-N-(3-Chlor-4-methoxybenzyl)-amid
   4-{3-[(3-Chlor-4-methoxybenzyl)-carbamoyl]-4,5-cyclopentenothiophen-2-ylcarbamoyl}-cyclohexancarbonsäuremethylester;
mit 2-Amino-4,5-cyclohepteno-thiophen-3-carbonsäure-N-(3-chlor-4-methoxybenzyl)-amid
   4-{3-[(3-Chlor-4-methoxybenzyl)-carbamoyl]-4,5-cycloheptenothiophen-2-ylcarbamoyl}-cyclohexancarbonsäuremethylester;
mit 2-Amino-5-ethyl-thiophen-3-carbonsäure-N-(3-chlor-4-methoxybenzyl)-amid
   4-{3-[(3-Chlor-4-methoxybenzyl)-carbamoyl]-5-ethyl-thiophen-2-yl-carbamoyl}-cyclohexancarbonsäuremethylester;
mit 2-Amino-4,5,6,7-tetrahydrobenzo[b]thiophen-3-carbonsäure-N-(3,4-dimethoxybenzyl)-amid
   4-{3-[(3,4-Dimethoxybenzyl)-carbamoyl]-4,5,6,7,-tetrahydrobenzo[b]thiophen-2-ylcarbamoyl}-cyclohexancarbonsäuremethylester;
mit 2-Amino-5-methyl-thiophen-3-carbonsäure-N-(3,4-dimethoxybenzyl)-amid
   4-{3-[(3,4-Dimethoxybenzyl)-carbamoyl]-5-methyl-thiophen-2-yl-carbamoyl}-cyclohexancarbonsäuremethylester;
mit 2-Amino-5-chlor-thiophen-3-carbonsäure-N-(3,4-dimethoxybenzyl)-amid
   4{3-[(3,4-Dimethoxybenzyl)-carbamoyl]-5-chlor-thiophen-2-yl-carbamoyl}-cyclohexancarbonsäuremethylester;
mit 2-Amino-4,5-cyclopenteno-thiophen-3-carbonsäure-N-(3,4-dimethoxybenzyl)-amid
   4-{3-[(3,4-Dimethoxybenzyl)-carbamoyl]-4,5-cyclopenteno-thiophen-2-ylcarbamoyl}-cyclohexancarbonsäuremethylester;
mit 2-Amino-4,5-cyclohepteno-thiophen-3-carbonsäure-N-(3,4-dimethoxybenzyl)-amid
   4-{3-[(3,4-Dimethoxybenzyl)-carbamoyl]-4,5-cyclohepteno-thiophen-2-ylcarbamoyl}-cyclohexancarbonsäuremethylester;
mit 2-Amino-5-ethyl-thiophen-3-carbonsäure-N-(3,4-dimethoxybenzyl)-amid
   4-{3-[(3,4-Dimethoxybenzyl)-carbamoyl]-5-ethyl-thiophen-2-yl-carbamoyl}-cyclohexancarbonsäuremethylester;
mit 2-Amino-4,5,6,7-tetrahydrobenzo[b]thiophen-3-carbonsäure-N-(4-fluorbenzyl)-amid
   4-{3-[(4-Fluorbenzyl)-carbamoyl]-4,5,6,7-tetrahydro-benzo[b]thiophen-2-ylcarbamoyl}-cyclohexancarbonsäuremethylester;
mit 2-Amino-5-methyl-thiophen-3-carbonsäure-N-(4-fluorbenzyl)-amid
   4-{3-[(4-Fluorbenzyl)-carbamoyl]-5-methyl-thiophen-2-ylcarbamoyl}-cyclohexancarbonsäuremethylester;
mit 2-Amino-5-chlor-thiophen-3-carbonsäure-N-(4-fluorbenzyl)-amid
   4-{3-[(4-Fluorbenzyl)-carbamoyl]-5-chlor-thiophen-2-ylcarbamoyl}-cyclohexancarbonsäuremethylester;
mit 2-Amino-4,5-cyclopenteno-thiophen-3-carbonsäure-N-(4-fluorbenzyl)-amid
   4-{3-[(4-Fluorbenzyl)-carbamoyl]-4,5-cyclopenteno-thiophen-2-yl-carbamoyl}-cyclohexancarbonsäuremethylester;
mit 2-Amino-4,5-cyclohepteno-thiophen-3-carbonsäure-N-(4-fluorbenzyl)-amid
   4-{3-[(4-Fluorbenzyl)-carbamoyl]-4,5-cyclohepteno-thiophen-2-yl-carbamoyl}-cyclohexancarbonsäuremethylester;
mit 2-Amino-5-ethyl-thiophen-3-carbonsäure-N-(4-fluorbenzyl)-amid
   4-{3-[(4-Fluorbenzyl)-carbamoyl]-5-ethyl-thiophen-2-ylcarbamoyl}-cyclohexancarbonsäuremethylester;
mit 2-Amino-4,5,6,7-tetrahydrobenzo[b]thiophen-3-carbonsäure-N-(3-nitrobenzyl)-amid
   4-{3-[(3-Nitrobenzyl)-carbamoyl]-4,5,6,7-tetrahydro-benzo[b]thiophen-2-ylcarbamoyl}-cyclohexancarbonsäuremethylester;
mit 2-Amino-5-methyl-thiophen-3-carbonsäure-N-(3-nitrobenzyl)-amid
   4-{3-[(3-Nitrobenzyl)-carbamoyl]-5-methyl-thiophen-2-ylcarbamoyl}-cyclohexancarbonsäuremethylester;
mit 2-Amino-5-chlor-thiophen-3-carbonsäure-N-(3-nitrobenzyl)-amid
   4-{3-[(3-Nitrobenzyl)-carbamoyl]-5-chlor-thiophen-2-ylcarbamoyl}-cyclohexancarbonsäuremethylester;
mit 2-Amino-4,5-cyclopenteno-thiophen-3-carbonsäure-N-(3-nitrobenzyl)-amid
   4-{3-[(3-Nitrobenzyl)-carbamoyl]-4,5-cyclopenteno-thiophen-2-yl-carbamoyl}-cyclohexancarbonsäuremethylester;
mit 2-Amino-4,5-cyclohepteno-thiophen-3-carbonsäure-N-(3-nitrobenzyl)-amid
   4-{3-[(3-Nitrobenzyl)-carbamoyl]-4,5-cyclohepteno-thiophen-2-yl-carbamol}-cyclohexancarbonsäuremethylester;
mit 2-Amino-5-ethyl-thiophen-3-carbonsäure-N-(3-nitrobenzyl)-amid
   4-{3-[(3-Nitrobenzyl)-carbamoyl]-5-ethyl-thiophen-2-ylcarbamoyl}-cyclohexancarbonsäuremethylester;
mit 2-Amino-4,5,6,7-tetrahydrobenzo[b]thiophen-3-carbonsäure-N-phenethylamid
   4-[3-(Phenethyl-carbamoyl)-4,5,6,7-tetrahydro-benzo[b]thiophen-2-yl-carbamoyl]-cyclohexancarbonsäuremethylester;
mit 2-Amino-5-methyl-thiophen-3-carbonsäure-N-phenethylamid
   4-[3-(Phenethyl-carbamoyl)-5-methyl-thiophen-2-ylcarbamoyl]-cyclo-hexancarbonsäuremethylester;
mit 2-Amino-5-chlor-thiophen-3-carbonsäure-N-phenethylamid
   4-[3-(Phenethyl-carbamoyl)-5-chlor-thiophen-2-ylcarbamoyl]-cyclohexancarbonsäuremethylester;
mit 2-Amino-4,5-cyclopenteno-thiophen-3-carbonsäure-N-phenethylamid
   4-[3-(Phenethyl-carbamoyl)-4,5-cyclopenteno-thiophen-2-yl-carbamoyl]- cyclohexancarbonsäuremethylester;
mit 2-Amino-4,5-cyclohepteno-thiophen-3-carbonsäure-N-phenethylamid
   4-[3-(Phenethyl-carbamoyl)-4,5-cyclohepteno-thiophen-2-yl-carbamoyl]- cyclohexancarbonsäuremethylester;
mit 2-Amino-5-ethyl-thiophen-3-carbonsäure-N-phenethylamid
   4-[3-(Phenethyl-carbamoyl)-5-ethyl-thiophen-2-ylcarbamoyl]- cyclohexancarbonsäuremethylester.

### Beispiel 3

Eine Lösung von (Benzo[1,3]-dioxol-5-ylmethyl)-amin in Dichlormethan und 1,1 Äquivalenten Pyridin wird mit 4-(3-Chlorocarbonyl-4,5,6,7-tetrahydro-benzo[bJthiophen-2-ylcarbamoyl)-benzoesäuremethylester versetzt und gerührt.
Das Lösungsmittel wird entfernt und wie üblich aufgearbeitet Man erhält 1,3 g 4-{3-[(Benzo[1,3]-dioxol-5-ylmethyl)-carbamoyl]-4,5,6,7-tetrahydrobenzo[b]thiophen-2-ylcarbamoyl}-benzoesäuremethylester, F. 165°.

### Beispiel 4

Eine Lösung von 1,3 g 4-{3-[(Benzo[1,3]-dioxol-5-ylmethyl)-carbamoyl]-4,5,6,7-tetrahydro-benzo[b]thiophen-2-ylcarbamoyl}-benzoesäuremethylester, 100 ml Methanol und 30 ml 1N NaOH wird 4 Stunden bei 50° gerührt. Man arbeitet wie üblich auf und erhält 4-{3-[(Benzo[1,3]-dioxol-5-ylmethyl)-carbamoyl]-4,5,6,7-tetrahydro-benzo[b]thiophen-2-ylcarbamoyl}-benzoesäure, F. 259-261°.

Analog erhält man durch Hydrolyse aus den in den Beispielen 1 und 2 erhaltenen Estern die nachstehenden Carbonsäuren:
4-{3-[(Benzo[1,3]-dioxol-5-ylmethyl)-carbamoyl]-5-methyl-thiophen-2-ylcarbamoyl}-benzoesäure, Hydrat, F. > 270°;
4-{3-[(Benzo[1,3]-dioxol-5-ylmethyl)-carbamoyl]-5,6-dimethylthiophen-2-ylcarbamoyl}-benzoesäure, Natriumsalz, amorph;
4-{3-[(Benzo[1,3]-dioxol-5-ylmethyl)-carbamoyl]-5-chlor-thiophen-2-ylcarbamoyl}-benzoesäure;
4-{3-[(Benzo[1,3]-dioxol-5-ylmethyl)-carbamoyl]-4,5-cyclopentenothiophen-2-ylcarbamoyl}-benzoesäure;
4-{3-[(Benzo[1,3]-dioxol-5-ylmethyl)-carbamoyl]-4,5-cycloheptenothiophen-2-ylcarbamoyl}-benzoesäure;
4-{3-[(Benzo[1,3]-dioxol-5-ylmethyl)-carbamoyl]-5-ethyl-thiophen-2-ylcarbamoyl}-benzoesäure, F. >270°;
4-{3-[(Benzo[1,3]-dioxol-5-ylmethyl)-carbamoyl]-5-propyl-thiophen-2-ylcarbamoyl}-benzoesäure;
4-{3-[(Benzo[1,3]-dioxol-5-ylmethyl)-carbamoyl]-5-isopropyl-thiophen-2-ylcarbamoyl}-benzoesäure, F. >270°;
4-{3-[(Benzo[1,3]-dioxol-5-ylmethyl)-carbamoyl]-5-butyl-thiophen-2-ylcarbamoyl}-benzoesäure, F. 245°;
4-[3-(Benzyl-carbamoyl)-4,5,6,7-tetrahydro-benzo[b]thiophen-2-yl-carbamoyl]-benzoesäure;
4-[3-(Benzyl-carbamoyl)-5-methyl-thiophen-2-ylcarbamoyl]-benzoesäure;
4-[3-(Benzyl-carbamoyl)-5-isopropyl-thiophen-2-ylcarbamoyl]benzoesäure, F. 275-277°;
4-[3-(Benzyl-carbamoyl)-5-chlor-thiophen-2-ylcarbamoyl-benzoesäure;
4-[3-(Benzyl-carbamoyl)-4,5-cyclopenteno-thiophen-2-ylcarbamoyl]-benzoesäure;
4-[3-(Benzyl-carbamoyl)-4,5-cyclohepteno-thiophen-2-ylcarbamoyl]-benzoesäure;
4-[3-(Benzyl-carbamoyl)-5-ethyl-thiophen-2-ylcarbamoyl]-benzoesäure;
4-{3-[(3-Chlor-4-methoxybenzyl)-carbamoyl]-4,5,6,7-tetrahydrobenzo[b]thiophen-2-ylcarbamoyl}-benzoesäure;
4-{3-[(3-Chlor-4-methoxybenzyl)-carbamoyl]-5-methyl-thiophen-2-yl-carbamoyl}-benzoesäure;
4-{3-[(3-Chlor-4-methoxybenzyl)-carbamoyl]-5-chlor-thiophen-2-yl-carbamoyl}-benzoesäure;
4-{3-[(3-Chlor-4-methoxybenzyl)-carbamoyl]-4,5-cyclopentenothiophen-2-ylcarbamoyl}-benzoesäure;
4-{3-[(3-Chlor-4-methoxybenzyl)-carbamoyl]-4,5-cycloheptenothiophen-2-ylcarbamoyl}-benzoesäure;
4-{3-[(3-Chlor-4-methoxybenzyl)-carbamoyl]-5-ethyl-thiophen-2-yl-carbamoyl}-benzoesäure;
4-{3-[(3,4-Dimethoxybenzyl)-carbamoyl]-4,5,6,7-tetrahydrobenzo[b]thiophen-2-ylcarbamoyl}-benzoesäure;
4-{3-[(3,4-Dimethoxybenzyl)-carbamoyl]-5-methyl-thiophen-2-yl-carbamoyl}-benzoesäure, Hydrat, F. > 270°;
4-{3-[(3,4-Dimethoxybenzyl)-carbamoyl]-5-chlor-thiophen-2-ylcarbamoyl}-benzoesäure;
4-{3-[(3,4-Dimethoxybenzyl)-carbamoyl]-4,5-cyclopenteno-thiophen2-ylcarbamoyl}-benzoesäure;
4-{3-[(3,4-Dimethoxybenzyl)-carbamoyl]-4,5-cyclohepteno-thiophen2-ylcarbamoyl}-benzoesäure;
4-{3-[(3,4-Dimethoxybenzyl)-carbamoyl]-5-ethyl-thiophen-2-ylcarbamoyl}-benzoesäure;
4-{3-[(4-Fluorbenzyl)-carbamoyl]-4,5,6,7-tetrahydrobenzo[b]thiophen-2-ylcarbamoyl}-benzoesäure;
4-{3-[(4-Fluorbenzyl)-carbamoyl]-5-methyl-thiophen-2-ylcarbamoyl}-benzoesäure;
4-{3-[(4-Fluorbenzyl)-carbamoyl]-5-chlor-thiophen-2-ylcarbamoyl}-benzoesäure;
4-{3-[(4-Fluorbenzyl)-carbamoyl]-4,5-cyclopenteno-thiophen-2-yl-carbamoyl}-benzoesäure;
4-{3-[(4-Fluorbenzyl)-carbamoyl]-4,5-cyclohepteno-thiophen-2-yl-carbamoyl}-benzoesäure;
4-{3-[(4-Fluorbenzyl)-carbamoyl]-5-ethyl-thiophen-2-ylcarbamoyl}-benzoesäure;
4-{3-[(3-Nitrobenzyl)-carbamoyl)-4,5,6,7-tetrahydro-benzo[b]thiophen-2-ylcarbamoyl}-benzoesäure;
4-{3-[(3-Nitrobenzyl)-carbamoyl]-5-methyl-thiophen-2-ylcarbamoyl}-benzoesäure;
4-{3-[(3-Nitrobenzyl)-carbamoyl]-5-chlor-thiophen-2-ylcarbamoylbenzoesäure;
4-{3-[(3-Nitrobenzyl)-carbamoyl]-4,5-cyclopenteno-thiophen-2-yl-carbamoyl}-benzoesäure;
4-{3-[(3-Nitrobenzyl)-carbamoyl]-4,5-cyclohepteno-thiophen-2-yl-carbamoyl}-benzoesäure;
4-{3-[(3-Nitrobenzyl)-carbamoyl]-5-ethyl-thiophen-2-ylcarbamoyl}-benzoesäure;
4-[3-(Phenethyl-carbamoyl)-4,5,6,7-tetrahydro-benzo[b]thiophen-2-yl-carbamoyl]-benzoesäure;
4-[3-(Phenethyl-carbamoyl)-5-methyl-thiophen-2-ylcarbamoyl]-benzoesäure;
4-[3-(Phenethyl-carbamoyl)-5-chlor-thiophen-2-ylcarbamoyl)-benzoesäure;
4-[3-(Phenethyl-carbamoyl)-4,5-cyclopenteno-thiophen-2-yl-carbamoyl]-benzoesäure;
4-[3-(Phenethyl-carbamoyl)-4,5-cyclohepteno-thiophen-2-yl-carbamoyl]-benzoesäure;
4-[3-(Phenethyl-carbamoyl)-5-ethyl-thiophen-2-ylcarbamoyl]-benzoesäure;
4-{3-[(Benzo[1,3]-dioxol-5-ylmethyl)-carbamoyl]-4,5,6,7-tetrahydrobenzo[b]thiophen-2-ylcarbamoyl}-cyclohexancarbonsäure, F. 265°.
4-{3-[(Benzo[1,3]-dioxol-5-ylmethyl)-carbamoyl]-5-methyl-thiophen-2-ylcarbamoyl}-cyclohexancarbonsäure, Natriumsalz, Dihydrat, F. 130°;
4-{3-[(Benzo[1,3]-dioxol-5-ylmethyl)-carbamoyl]-5,6-dimethylthiophen-2-ylcarbamoyl}-cyclohexancarbonsäure;
4-{3-[(Benzo[1,3]-dioxol-5-ylmethyl)-carbamoyl]-5-chlor-thiophen-2-ylcarbamoyl}-cyclohexancarbonsäure;
4-{3-[(Benzo[1,3]-dioxol-5-ylmethyl)-carbamoyl]-4,5-cyclopentenothiophen-2-ylcarbamoyl}-cyclohexancarbonsäure;
4-{3-[(Benzo[1,3]-dioxol-5-ylmethyl)-carbamoyl]-4,5-cycloheptenothiophen-2-ylcarbamoyl}-cyclohexancarbonsäure;
4-{3-[(Benzo[1,3]-dioxol-5-ylmethyl)-carbamoyl]-5-ethyl-thiophen-2-ylcarbamoyl}-cyclohexancarbonsäure, Natriumsalz, Trihydrat, F. 133°;
4-[3-(Benzyl-carbamoyl)-4,5,6,7-tetrahydro-benzo[b]thiophen-2-yl-carbamoyl]-cyclohexancarbonsäure;
4-[3-(Benzyl-carbamoyl)-5-methyl-thiophen-2-ylcarbamoyl]-cyclohexancarbonsäure, F. 266°;
4-[3-(Benzyl-carbamoyl)-5-chlor-thiophen-2-ylcarbamoyl]-cyclohexancarbonsäure;
4-[3-(Benzyl-carbamoyl)-4,5-cyclopenteno-thiophen-2-ylcarbamoyl]-cyclohexancarbonsäure;
4-[3-(Benzyl-carbamoyl)-4,5-cyclohepteno-thiophen-2-ylcarbamoyl]-cyclohexancarbonsäure;
4-[3-(Benzyl-carbamoyl)-5-ethyl-thiophen-2-ylcarbamoyl]-cyclo-hexancarbonsäure;
4-{3-[(3-Chlor-4-methoxybenzyl)-carbamoyl]-4,5,6,7-tetrahydrobenzo[b]thiophen-2-ylcarbamoyl}-cyclohexancarbonsäure;
4-{3-[(3-Chlor-4-methoxybenzyl)-carbamoyl]-5-methyl-thiophen-2-yl-carbamoyl}-cyclohexancarbonsäure;
4-{3-[(3-Chlor-4-methoxybenzyl)-carbamoyl]-5-chlor-thtophen-2-yl-carbamoyl}-cyclohexancarbonsäure;
4-{3-[(3-Chlor-4-methoxybenzyl)-carbamoyl]-4,5-cyclopentenothiophen-2-ylcarbamoyl}-cyclohexancarbonsäure;
4-{3-[(3-Chlor-4-methoxybenzyl)-carbamoyl]-4,5-cycloheptenothiophen-2-ylcarbamoyl}-cyclohexancarbonsäure;
4-{3-[(3-Chlor-4-methoxybenzyl)-carbamoyl]-5-ethyl-thiophen-2-yl-carbamoyl}-cydohexancarbonsäure;
4-{3-[(3,4-Dimethoxybenzyl)-carbamoyl]-4,5,6,7-tetrahydrobenzo[b]thiophen-2-ylcarbamoyl}-cyclohexancarbonsäure;
4-{3-[(3,4-Dimethoxybenzyl)-carbamoyl]-5-methyl-thiophen-2-yl-carbamoyl}-cyclohexancarbonsäure;
4-{3-[(3,4-Dimethoxybenzyl)-carbamoyl]-5-chlor-thiophen-2-yl-carbamoyl}-cyclohexancarbonsäure;
4-{3-[(3,4-Dimethoxybenzyl)-carbamoyl]-4,5-cyclopenteno-thiophen-2-ylcarbamoyl}-cyclohexancarbonsäure;
4-{3-[(3,4-Dimethoxybenzyl)-carbamoyl]-4,5-cyclohepteno-thiophen-2-ylcarbamoyl}-cyclohexancarbonsäure;
4-{3-[(3,4-Dimethoxybenzyl)-carbamoyl]-5-ethyl-thiophen-2-yl-carbamoyl}-cyclohexancarbonsäure;
4-{3-[(4-Fluorbenzyl)-carbamoyl]-4,5,6,7-tetrahydrobenzo[b]thiophen-2-ylcarbamoyl}-cyclohexancarbonsäure;
4-{3-[(4-Fluorbenzyl)-carbamoyl]-5-methyl-thiophen-Z-ylcarbamoyl]-cyclohexancarbonsäure;
4-(3-[(4-Fluorbenzyl)-carbamoyl]-5-chlor-thiophen-2-ylcarbamoyl}-cyclohexancarbonsäure;
4-{3-[(4-Fluorbenzyl)-carbamoyl]-4,5-cyclopenteno-thiophen-2-yl-carbamoyl}-cyclohexancarbonsäure;
4-{3-[(4-Fluorbenzyl)-carbamoyl]-4,5-cyclohepteno-thiophen-2-yl-carbamoyl}-cyclohexancarbonsäure;
4-{3-[(4-Fluorbenzyl)-carbamoyl]-5-ethyl-thiophen-2-ylcarbamoyl}-cydohexancarbonsäure;
4-{3-[(3-Nitrobenzyl)-carbamoyl]-4,5,6,7,-tetrahydro-benzo[b]thiophen-2-ylcarbamoyl}-cyclohexancarbonsäure;
4-{3-[(3-Nitrobenzyl)-carbamoyl]-5-methyl-thiophen-2-ylcarbamoyl}-cyclohexancarbonsäure;
4-{3-[(3-Nitrobenzyl)-carbamoyl]-5-chlor-thiophen-2-ylcarbamoyl}-cyclohexancarbonsäure;
4-{3-[(3-Nitrobenzyl)-carbamoyl]-4,5-cyclopenteno-thiophen-2-yl-carbamoyl}-cyclohexancarbonsäure;
4-{3-[(3-Nitrobenzyl)-carbamoyl]-4,5-cyclohepteno-thiophen-2-yl-carbamoyl}-cyclohexancarbonsäure;
4-{3-[(3-Nitrobenzyl)-carbamoyl]-5-ethyl-thiophen-2-ylcarbamoyl}-cyclohexancarbonsäure;
4-[3-(Phenethyl-carbamoyl)-4,5,6,7-tetrahydro-benzo[b]thiophen-2-yl-carbamoyl]-cyclohexancarbonsäure
4-[3-(Phenethyl-carbamoyl)-5-methyl-thlophen-2-ylcarbamoyl]-cyclohexancarbonsäure;
4-[3-(Phenethyl-carbamoyl)-5-chlor-thiophen-2-ylcarbamoyl]-cyclohexancarbonsäure;
4-[3-(Phenethyl-carbamoyl)-4,5-cyclopenteno-thiophen-2-yl-carbamoyl]- cyclohexancarbonsäure;
4-[3-(Phenethyl-carbamoyl)-4,5-cyclohepteno-thiophen-2-yl-carbamoyl]- cyclohexancarbonsäure;
4-[3-(Phenethyl-carbamoyl)-5-ethyl-thiophen-2-ylcarbamoyl]-cyclohexancarbonsäure.

Analog erhält man nachstehende Verbindungen
4-[3-(Benzyl-carbamoyl)-5-isopropyl-thiophen-2-ylcarbamoyl]-cyclohexancarbonsäure, F. 198°;
4-[3-(Benzyl-carbamoyl)-5-propyl-thiophen-2-ylcarbamoyl]-benzoesäure, F. 268°;
4-{3-[(Benzo[1,3]-dioxol-5-ylmethyl)-carbamoyl]-5-isopropyl-thiophen-2-ylcarbamoyl}-cyclohexancarbonsäure, Natriumsalz, F. 240°.

### Beispiel 5

Eine Lösung von 4-{3-[(3-Nitrobenzyl)-carbamoyl]-5-ethyl-thiophen-2-yl-carbamoyl}-cyclohexancarbonsäurein Methanol wird in Gegenwart von Raney-Nickel hydriert. Der Katalysator wird abfiltriert und die Lösung eingeengt. Man erhält nach Umkristallisation 4-{3-[(3-Aminobenzyl)-carbamoyl]-5-ethyl-thiophen-2-ylcarbamoyl}-cyclohexancarbonsäure.

### Beispiel 6

Analog Beispiel 1 erhält man durch Umsetzung von 2-Amino-4,5,6,7-tetrahydrobenzo[b]thiophen-3-carbonsäure-(benzo[1,3]dioxol-5-ylmethyl)-amid und 3-Nitrobenzoylchlorid die Verbindung 4-{3-[(Benzo[1,3]-dioxol-5-ylmethyl)-carbamoyl]-4,5,6,7-tetrahydro-benzo[b]thiophen-2-ylcarbamoyl}-3-nitrobenzol.

Analog erhält man 4-{3-[(3,4-Dimethoxybenzyl)-carbamoyl]-5-methylthiophen-2-ylcarbamoyl}-3-nitrobenzol.

Analog Beispiel 5 erhält man durch katalytische Reduktion aus den 3-Nitro-derivaten die nachstehenden Verbindungen
4-{3-[(Benzo[1,3]-dioxol-5-ylmethyl)-carbamoyl]-4,5,6,7-tetrahydrobenzo[b]thiophen-2-ylcarbamoyl}-3-aminobenzol und
4-{3-[(3,4-Dimethoxybenzyl)-carbamoyl]-5-methyl-thiophen-2-yl carbamoyl}-3-aminobenzol.

Durch Umsetzung mit äquivalenten Mengen Methylsulfonylchlorid und Pyridin in Dichlormethan erhält man
4-{3-[(Benzo[1,3]-dioxol-5-ylmethyl)-carbamoyl]-4,5,6,7-tetrahydrobenzo[b]thiophen-2-ylcarbamoyl}-3-methylsulfonamidobenzol und
4-{3-[(3,4-Dimethoxybenzyl)-carbamoyl]-5-methyl-thiophen-2-yl carbamoyl}-3-methylsulfonamidobenzol.

### Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3I zweifach destilliertem Wasser mit 2 n Salzsäure auf pH 6,5 eingestellt, steril filtriert in Injektionsgläser abgefüllt. unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄. 2 H₂O, 28,48 g Na₂HPO₄. 12 H₂O und 0,1 g Benzalkoniumchlorid in 940 ml zweifach destilliertem Wasser. Man stellt auf pH 6,8 ein, füllt auf 11 auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält

### Beispiel H: Ampullen

Eine Lösung von 1 kg Wirkstoff der Formel I in 60 zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 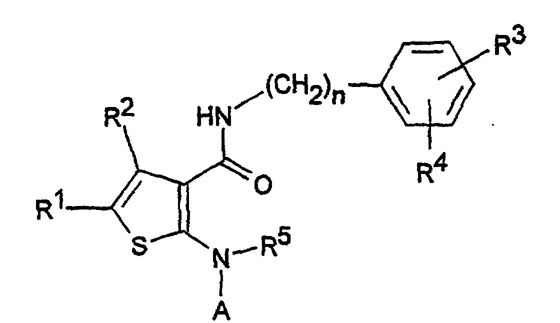 worin
R¹, R² jeweils unabhängig voneinander H, A, OA, Alkenyl, Alkinyl, CF₃ oder Hal,
wobei einer der Reste R¹ oder R² immer ≠ H ist,
R¹ und R² zusammen auch Alkylen mit 3-5 C-Atomen,
R³, R⁴ jeweils unabhängig voneinander H, A, OA, NO₂, NH₂ oder Hal,
R³ und R⁴ zusammen auch -O-CH₂-CH₂-, -O-CH₂-O- oder -O-CH₂-CH₂-O-,
A, A' jeweils unabhängig voneinander H oder Alkyl mit 1 bis 6 C-Atomen,
R⁵ -X-Y,
X CO, CS oder SO₂,
Y einen unsubstituierten oder ein- oder zweifach durch COOH, COOA, CONH₂, CONAA', CONHA, CN, NHSO₂A, N(SO₂A)₂ oder SO₂A substituierten gesättigten oder ungesättigten 5-7-gliedrigen isocyclischen Ring,
Hal F, Cl, Br oder I
und
n 0, 1, 2 oder 3
bedeuten, sowie deren physiologisch unbedenklichen Salze,
wobei 2-Benzoylamino-4,5,6,7-tetrahydro-benzo[b]thiophen-3-carbonsäure N-phenylamid ausgeschlossen ist.

2. Verbindungen der Formel I gemäß Anspruch 1
(a) 4-{3-[(Benzo[1,3]-dioxol-5-ylmethyl)-carbamoyl}-4,5,6,7-tetrahydro-benzo[b]thiophen-2-ylcarbamoyl}-benzoesäure;
(b) 4-[3-(Benzylcarbamoyl)-5-methyl-thiophen-2-ylcarbamoyl]-benzoesäure;
(c) 4-{3-[(Benzo[1,3]-dioxol-5-ylmethyl)-carbamoyl}-5-methylthiophen-2-ylcarbamoyl}-benzoesäure;
(d) 4-{3-[(Benzo[1,3]-dioxol-5-ylmethyl)-carbamoyl}-5-methylthiophen-2-ylcarbamoyl}-cyclohexancarbonsäure;
(e) 4-{3-[(Benzo[1,3]-dioxol-5-ylmethyl)-carbamoyl}-4,5,6,7-tetrahydro-benzo[b]thiophen-2-ylcarbamoyl}-benzoesäure;
(f) 4-{3-[(3-Chlor-4-methoxybenzyl)-carbamoyl]-4,5,6,7-tetrahydrobenzo[b]thiophen-2-ylcarbamoyl}benzoesäure;
(g) 4-{3-[(3-Chlor-4-methoxybenzyl)-carbamoyl]-5-methyl-thiophen-2-ylcarbamoyl}-benzoesäure;
(h) 4-{3-[(3-Chlor-4-methoxybenzyl)-carbamoyl]-5-ethyl-thiophen-2-ylcarbamoyl}-benzoesäure;
(i) 4-{3-[(3-Chlor-4-methoxybenzyl)-carbamoyl]-4,5,6,7-tetrahydrobenzo[b]thiophen-2-ylcarbamoyl}-cyclohexancarbonsäure;
(k) 4-{3-[(3-Chlor-4-methoxybenzyl)-carbamoyl]-5-methyl-thiophen-2-ylcarbamoyl}-cyclohexancarbonsäure
sowie deren physiologisch unbedenklichen Salze.

3. Verfahren zur Herstellung von Verbindungen der Formel I nach Anspruch 1 sowie deren Salzen,
dadurch gekennzeichnet, daß man
a) eine Verbindung der Formel II 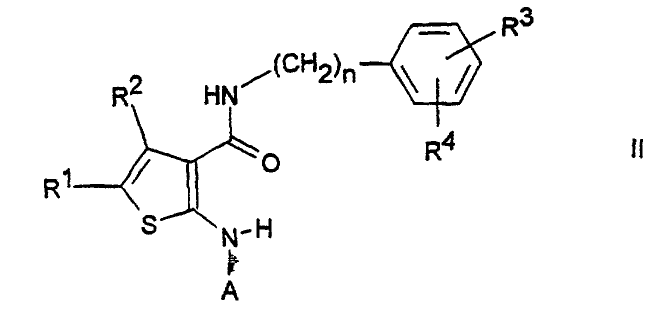 worin
R¹, R², R³, R⁴ und n die angegebenen Bedeutungen haben,
mit einer Verbindung der Formel III
L-R⁵ III
umsetzt, worin R⁵ die angegebene Bedeutung hat und
L Cl, Br, I, OH oder eine reaktionsfähig veresterte OH-Gruppe bedeutet,
oder
b) eine Verbindung der Formel IV 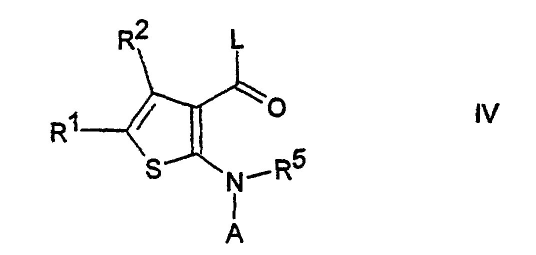 worin
R¹, R², R⁵ und A die angegebenen Bedeutungen haben,
und L Cl, Br, 1, OH oder eine reaktionsfähig veresterte OH-Gruppe bedeutet,
mit einer Verbindung der Formel V 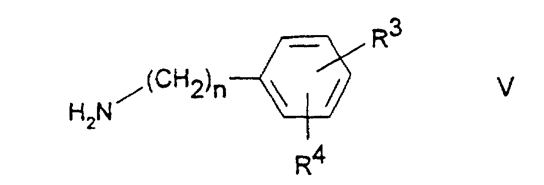 worin
R³, R⁴ und n die angegebenen Bedeutungen haben,
umsetzt,
oder
c) in einer Verbindung der Formel I einen Rest R³, R⁴ und/oder
R⁵ in einen anderen Rest R³, R⁴ und/oder R⁵ umwandelt, indem man einen Ester verseift oder eine Nitrogruppe reduziert,
und/oder daß man eine saure Verbindung der Formel I durch Behandeln mit einer Base in eines ihrer Salze überführt.

4. Verfahren zur Herstellung pharmazeutischer Zubereitungen, dadurch gekennzeichnet, daß man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Krankheiten des Herz-Kreislaufsystems und zur Therapie von Potenzstörungen.

7. Arzneimittel der Formel I nach Anspruch 1 und ihre physiologisch unbedenklichen Salze als Phosphodiesterase V-Hemmer.

8. Verwendung von Verbindungen der Formel I nach Anspruch 1 und/oder ihre physiologisch unbedenklichen Salze zur Herstellung eines Arzneimittels.

9. Verwendung von Verbindungen der Formel I nach Anspruch 1 und/oder ihrer physiologisch unbedenklichen Salze bei der Bekämpfung von Krankheiten.

## Claims

1. Compounds of the formula I 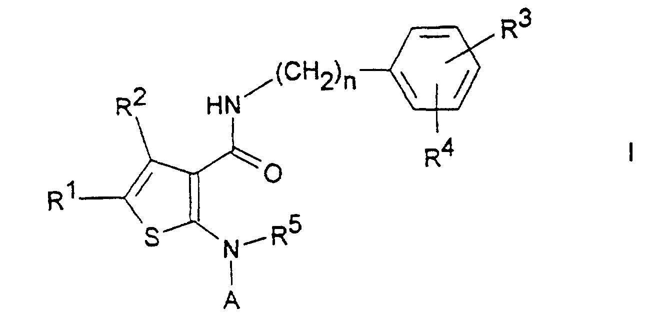 where
R¹ and R², independently of one another, are each H, A, OA, alkenyl, alkynyl, CF₃ or Hal, one of the radicals R¹ or R² always being ≠ H,
R¹ and R² together are also alkylene having 3-5 C atoms,
R³ and R⁴, independently of one another, are each H, A, OA, NO₂, NH₂ or Hal,
R³ and R⁴ together are also -O-CH₂-CH₂-, -O-CH₂-O- or -O-CH₂-CH₂-O-,
A and A', independently of one another, are each H or alkyl having 1 to 6 C atoms,
R⁵ is -X-Y,
X is CO, CS or SO₂,
Y is a saturated or unsaturated 5- to 7-membered isocyclic ring which is unsubstituted or monosubstituted or disubstituted by COOH, COOA, CONH₂, CONAA', CONHA, CN, NHSO₂A, N(SO₂A)₂ or SO₂A,
Hal is F, Cl, Br or I
and
n is 0, 1, 2 or 3,
and their physiologically acceptable salts,
with N-phenyl-2-benzoyl-4,5,6,7-tetrahydrobenzo[b] thiophene-3-carboxamide being excluded.

2. Compounds of the formula I according to Claim 1
(a) 4-{3- [(benzo[1,3]dioxol-5-ylmethyl)carbamoyl]-4,5,6,7-tetrahydrobenzo[b]thiophen-2-ylcarbamoyl}-benzoic acid;
(b) 4- [3- (benzylcarbamoyl)-5-methylthiophen-2-ylcarbamoyl]benzoic acid;
(c) 4-{3-[(benzo [1,3]dioxol-5-ylmethyl)carbamoyl]-5-methylthiophen-2-ylcarbamoyl}benzoic acid;
(d) 4-{3-[(benzo[1,3]dioxol-5-ylmethyl)carbamoyl]-5-methylthiophen-2-ylcarbamoyl}cyclohexanecarboxylic acid;
(e) 4-{3-[(benzo[1,3] dioxol-5-ylmethyl)carbamoyl]-4,5,6,7-tetrahydrobenzo[b]thiophen-2-ylcarbamoyl}-benzoic acid;
(f) 4-{3-[(3-chloro-4-methoxybenzyl)carbamoyl]-4,5,6,7-tetrahydrobenzo [b] thiophen-2-ylcarbamoyl}-benzoic acid;
(g) 4-(3-[(3-chloro-4-methoxybenzyl) carbamoyl]-5-methylthiophen-2-ylcarbamoyl}benzoic acid;
(h) 4-{3-[(3-chloro-4-methoxybenzyl)carbamoyl]-5-ethylthiophen-2-ylcarbamoyl}benzoic acid;
(i) 4-{3-[(3-chloro-4-methoxybenzyl) carbamoyl]-4,5,6,7-tetrahydrobenzo[b]thiophen-2-ylcarbamoyl}-cyclohexanecarboxylic acid;
(k) 4-{3- [(3-chloro-4-methoxybenzyl)carbamoyl]-5-methylthiophen-2-ylcarbamoyl}-cyclohexanecarboxylic acid and their physiologically acceptable salts.

3. Process for the preparation of compounds of the formula I according to Claim 1 and their salts, characterized in that
a) a compound of the formula II 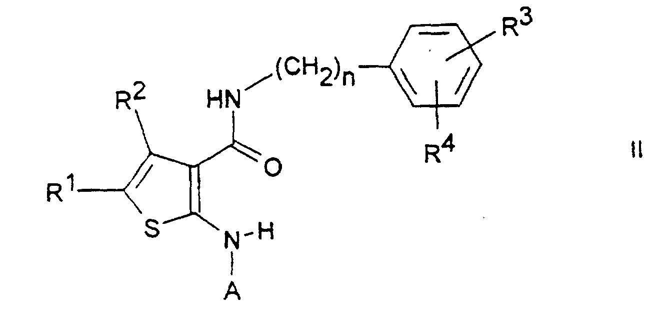 wherein
R¹, R², R³, R⁴ and n have the stated meanings,
is reacted with a compound of the formula III
L-R⁵ III
wherein R⁵ has the stated meaning and
L is Cl, Br, I, OH or an OH group rendered reactive by esterification,
or
b) a compound of the formula IV 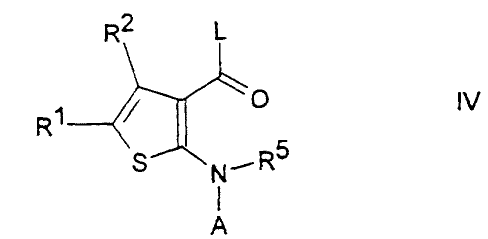 wherein
R¹, R², R⁵ and A have the stated meanings
and L is Cl, Br, I, OH or an OH group rendered reactive by esterification,
is reacted with a compound of the formula V 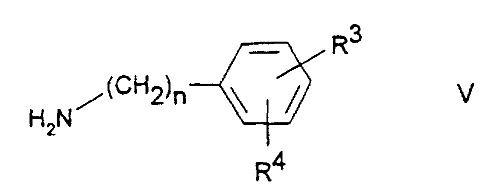 wherein
R³, R⁴ and n have the stated meanings,
or
c) in a compound of the formula I, a radical R³, R⁴ and/or R⁵ is converted into another radical R³, R⁴ and/or R⁵ by hydrolysing an ester or reducing a nitro group,
and/or that an acidic compound of the formula I is converted into one of its salts by treatment with a base.

4. Process for the preparation of pharmaceutical formulations, characterized in that a compound of the formula I according to Claim 1 and/or one of its physiologically acceptable salts, together with at least one solid, liquid or semiliquid vehicle or excipient, is brought into a suitable dosage form.

5. Pharmaceutical formulation characterized by a content of at least one compound of the formula I according to Claim 1 and/or one of its physiologically acceptable salts.

6. Compounds of the formula I according to Claim 1 and their physiologically acceptable salts for the treatment of diseases of the cardiovascular system and for the therapy of disturbances of potency.

7. Drugs of the formula I according to Claim 1 and their physiologically acceptable salts as phosphodiesterase V inhibitors.

8. Use of compounds of the formula I according to Claim 1 and/or their physiologically acceptable salts for the preparation of a drug.

9. Use of compounds of the formula I according to Claim 1 and/or their physiologically acceptable salts in the treatment of diseases.

## Revendications

1. Les composés de formule I : 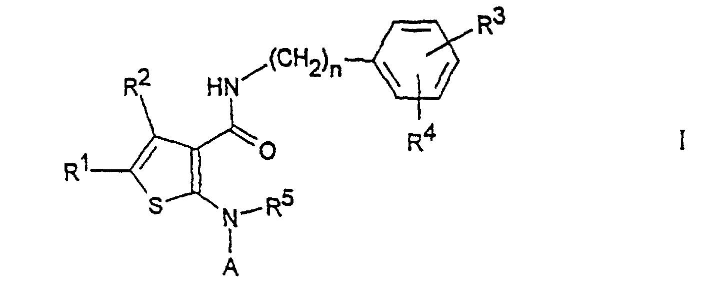 où
R¹, R² représentent, indépendamment l'un de l'autre, H, A, OA, un alcényle, un alcynyle, CF₃ ou Hal, l'un des restes R¹ ou R² étant toujours différent de H,
R¹ et R² représentent ensemble également un alkylène comportant 3 à 5 atomes de C,
R³, R⁴ représentent, indépendamment l'un de l'autre, H, A, OA, NO₂, NH₂ ou Hal,
R³ et R⁴ représentent ensemble également -O-CH₂-CH₂-, -O-CH₂-O- ou -O-CH₂-CH₂-O-,
A, A' représentent, indépendamment l'un de l'autre, H ou un alkyle comportant 1 à 6 atomes de C,
R⁵ représente -X-Y,
X CO, CS ou SO₂,
Y un cycle isocyclique ayant 5 à 7 chaînons, saturé ou insaturé, non substitué ou mono ou disubstitué par COOH, COOA, CONH2, CONAA', CONHA, CN, NHSO₂A, N(SO₂A)₂ ou SO₂A,
Hal F, Cl, Br ou I, et
n est égal à 0, 1, 2 ou 3,
ainsi que leurs sels physiologiquement acceptables, le N-phénylamide de l'acide 2-benzoylamino-4,5,6,7-tétrahydrobenzo[b]thiophène-3-carboxylique étant exclu.

2. Les composés de formule I selon la revendication 1
(a) l'acide 4-{3-[(benzo[1,3]dioxol-5-ylméthyl)-carbamoyl]-4,5,6,7-tétrahydrobenzo[b]thiophén-2-ylcarbamoyl}-benzoïque,
(b) l'acide 4-[3-(benzylcarbamoyl)-5-méthylthiophén-2]-ylcarbamoylbenzoïque,
(c) l'acide 4-{3-[(benzo[1,3]dioxol-5-ylméthyl)-carbamoyl]-5-méthylthiophén-2-ylcarbamoyl}-benzoïque,
(d) l'acide 4-{3-[(benzo[1,3]dioxol-5-ylméthyl)-carbamoyl]-5-méthylthiophén-2-ylcarbamoyl}-cyclohexanoïque,
(e) l'acide 4-{3-[(benzo[1,3]dioxol-5-ylméthyl)-carbamoyl]-4,5,6,7-tétrahydrobenzo[b]thiophén-2-ylcarbamoyl}-benzoïque,
(f) l'acide 4-{3-[(3-chloro-4-méthoxybenzyl)-carbamoyl]-4,5,6,7-tétrahydrobenzo[b]thiophén-2-ylcarbamoyl]-benzoïque,
(g) l'acide 4-{3-[(3-chloro-4-méthoxybenzyl)-carbamoyl]-5-méthylthiophén-2-ylcarbamoyl}-benzoïque,
(h) l'acide 4-{3-[(3-chloro-4-méthoxybenzyl)-carbamoyl]-5-éthylthiophén-2-ylcarbamoyl}-benzoïque,
(i) l'acide 4-{3-[(3-chloro-4-méthoxybenzyl)-carbamoyl]-4,5,6,7-tétrahydrobenzo[b]thiophén-2-ylcarbamoy}-cyclohexanoïque,
(k) l'acide 4-(3-[(3-chloro-4-méthoxybenzyl)-carbamoyl]-5-méthylthiophén-2-ylcarbamoyl}-cyclohexanoïque,
ainsi que leurs sels physiologiquement acceptables.

3. Procédé pour la préparation des composés de formule I selon la revendication 1, ainsi que de leurs sels, caractérisé en ce que :
a) l'on fait réagir un composé de formule II : 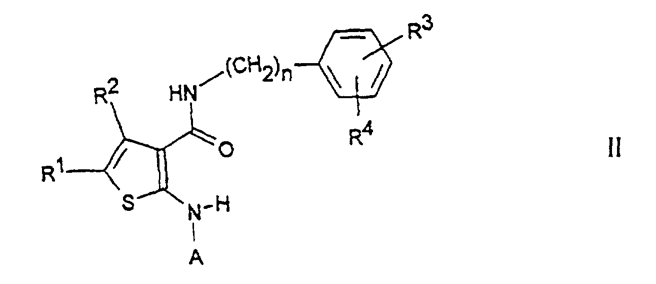 où
R¹, R², R³, R⁴ et n ont les significations indiquées,
avec un composé de formule III:
L-R⁵ III
où R⁵ a la signification indiquée et
L représente Cl, Br, I, OH ou un groupe OH estérifié réactif ou
b) l'on fait réagir un composé de formule IV : 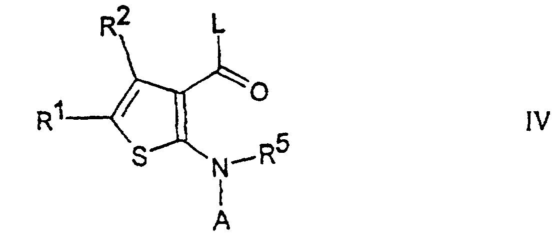 où
R¹, R², R⁵ et A ont les significations indiquées, et
L représente Cl, Br, 1, OH ou un groupe OH estérifié réactif,
avec un composé de formule V : 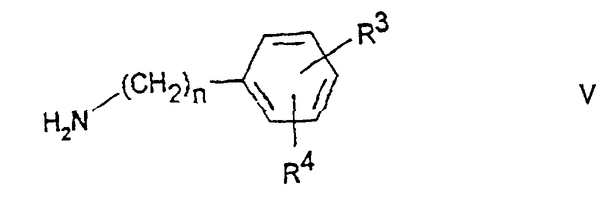 où
R³, R⁴ et n ont les significations indiquées, ou
c) l'on transforme dans un composé de formule I un reste R³, R⁴ et/ou R⁵ en un autre reste R³, R⁴ et/ou R⁵, en saponifiant un ester ou en réduisant un groupe nitro,
et/ou l'on transforme un composé acide de formule I en l'un de ses sels par traitement avec une base.

4. Procédé pour la fabrication de préparations pharmaceutiques, caractérisé en ce que l'on met sous la forme d'un dosage approprié un composé de formule I selon la revendication 1 et/ou l'un de ses sels physiologiquement acceptables, associé à au moins un support ou un adjuvant solide, liquide ou semi-liquide.

5. Préparation pharmaceutique, caractérisée en ce qu'elle contient au moins un composé de formule I selon la revendication 1 et/ou l'un de ses sels physiologiquement acceptables.

6. Composés de formule I selon la revendication 1 et leurs sels physiologiquement acceptables pour lutter contre les maladies du système de circulation cardiaque et à la thérapie des troubles de la puissance sexuelle.

7. Médicament de formule I selon la revendication 1 et leurs sels physiologiquement acceptables comme inhibiteur de la phosphodiestérase V.

8. Utilisation des composés de formule I selon la revendication 1 et/ou de leurs sels physiologiquement acceptables pour la fabrication d'un médicament.

9. Utilisation des composés de formule I selon la revendication 1 et/ou de leurs sels physiologiquement acceptables pour lutter contre les maladies.
